# EUROPÄISCHE PATENTSCHRIFT

(11) **EP 0 630 610 B1**
(45) Veröffentlichungstag und Bekanntmachung des Hinweises auf die Patenterteilung: **27.10.1999**
(21) Anmeldenummer: 94100148.9
(22) Anmeldetag: 07.01.1994
(51) Int. Cl.: A61B 6/08

(54) **Vorrichtung zur Anzeige einer Bewegung eines Patienten**
Device for monitoring the movement of a patient
Dispositif pour surveiller le mouvement d'un patient

(30) Priorität: 15.06.1993 DE 9308917 U
(43) Veröffentlichungstag der Anmeldung: 28.12.1994
(73) Patentinhaber: LAP GmbH Laser Applikationen, D-21337 Lüneburg (DE)
(72) Erfinder: Röckseisen, Armin Dr., D-21379 Scharnebeck (DE)
(74) Vertreter: Patentanwälte Hauck, Graalfs, Wehnert, Döring, Siemons

(56) Entgegenhaltungen:
- EP-A- 0 530 596
- DE-A- 3 803 566
- FR-A- 2 384 481
- GB-A- 2 112 172

## Beschreibung

Die Erfindung bezieht sich auf eine Vorrichtung zur Anzeige einer Bewegung eines Patienten nach dem Oberbegriff des Anspruchs 1.

Für die medizinische Diagnostik mit Strahlen oder die Strahlentherapie ist es wichtig, daß der Patient eine ruhige Lage beibehält. Andernfalls werden falsche Organstellen behandelt oder eine Untersuchung muß wiederholt werden.

Aus EP-A-530 596 ist eine Vorrichtung zum Messen der elektromagnetischen Abstrahlung des Kopfes eines Patienten bekanntgeworden, bei der der Patient auf einer Liege in einem Gehäuse gelagert wird, in das hinein ein Lichtleiter geführt ist, der am kopfseitigen Ende des Tisches befestigt ist. Der Lichtleiter wird von einer lichtemittierenden Diode impulsweise mit Licht versorgt, das auf den Kopf des Patienten gerichtet ist. Am Kopf wird ein Retro-Reflektorelement angebracht. Ein weiterer Lichtleiter empfängt das vom Retro-Reflektorelement erhaltene Licht und leitet es zu einer Fotozelle. Ein Grenzdetektor stellt fest, wenn das auf die Fotozelle auftreffende Licht eine untere Grenze unterschreitet.

Aus GB-A-2 112 172 ist bekannt, mit Hilfe von Lasern Linienstrahlen zu erzeugen, die in einem Behandlungsraum installiert sind und mit deren Hilfe eine Patientenpositionierung erfolgt.

Der Erfindung liegt die Aufgabe zugrunde, eine Vorrichtung zu schaffen, mit der die Bewegung eines Körpers und eines Körperteils eines Patienten mit einfachen Mitteln automatisch überwacht werden kann, wenn der Körper oder das Körperteil eine Therapie oder einer Diagnose unterworfen wird.

Diese Aufgabe wird durch die Merkmale des Anspruchs 1 gelöst.

Die erfindungsgemäße Vorrichtung enthält eine Laserquelle in Form eines modulierten Halbleiterlasers, deren Strahl linienförmig ist. Ferner wird eine optoelektronische Empfangsvorrichtung vorgesehen, die den reflektierten Laserstrahl empfängt und ein entsprechendes Signal erzeugt. Die Reflexion des Laserstrahls erfolgt durch ein am Patienten anbringbares Retro-Reflektorelement. Mit der Empfangsvorrichtung ist außerdem eine Anzeige und/oder Alarmvorrichtung verbunden zur Erzeugung eines Anzeige- oder Alarmsignals, wenn der reflektierte Strahl ausbleibt.

Da der Laserstrahl nur einen geringen Querschnitt hat, ist auch der reflektierte Strahl scharf begrenzt, so daß bereits geringe Auslenkungen des Reflektorelements von einer Standardrichtung von einer geeigneten optischen Empfangsvorrichtung erfaßt werden können.

Bei der Erfindung wird davon ausgegangen, daß es relativ einfach ist, ein Retro-Reflektorelement an einen Körperteil des Patienten anzubringen. Wegen der geringen Querausdehnung eines Laserstrahls braucht es nur eine kleine Fläche zu umfassen und kann daher auch innerhalb des Bereiches angeordnet werden, der zum Beispiel einer Strahlenbehandlung oder -diagnose unterworfen ist.

Das Reflektorelement kann mit geeigneten Mitteln zur Anbringung am Patienten ausgestattet werden. Vorzugsweise weist es eine Haftkleberschicht an seiner Rückseite auf Das Reflektorelement ist vorzugsweise ein Wegwerfartikel, der nach Benutzung nicht wieder verwendet wird. Es ist jedoch auch ohne weiteres denkbar, einen wiederverwendbaren Reflektor vorzusehen. Wird die erfindungsgemäße Vorrichtung in Verbindung mit einem Bestrahlungs- oder Diagnosegerät verwendet, kann es sinnvoll sein, sie in das Bestrahlungs- oder Diagnosegerät zu integrieren. Laser und Empfangsvorrichtung sind in einem gemeinsamen Gehäuse aufgenommen.

Kommt es zu einer Bewegung des Patienten, erzeugt die Anzeige und/oder Alarmvorrichtung ein entsprechendes optisches und/oder akustisches Signal. Ferner kann auch ein elektrisches Signal erzeugt werden, das zum Beispiel für eine Unterbrechung der Strahlenbehandlung oder Diagnose sorgt.

Die Erfindung wird nachfolgend anhand eines Ausführungsbeispiels näher erläutert.

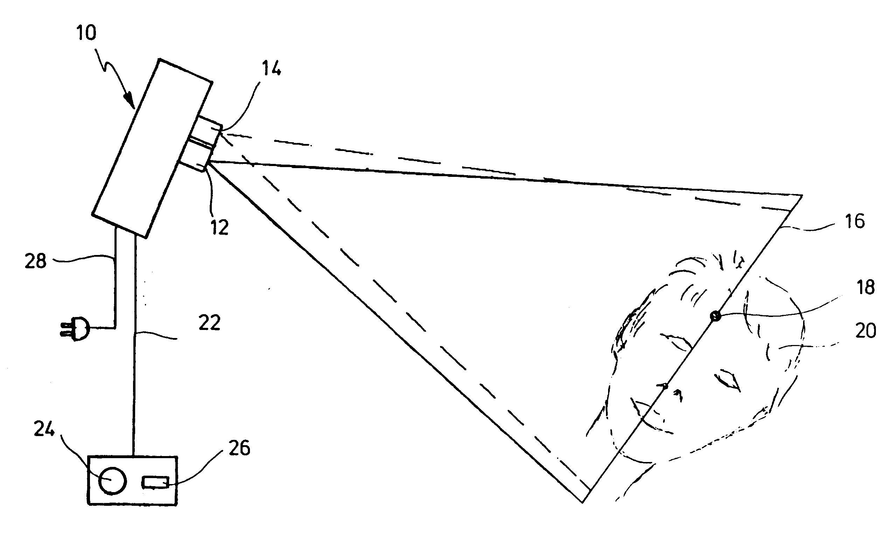

Die einzige Figur zeigt äußerst schematisch eine Vorrichtung nach der Erfindung.

In einem Gehäuse 10 ist eine Laserlichtquelle 12 angeordnet, die einen linienförmigen Strahl, wie bei 16 angedeutet, erzeugt. Neben der Laserlichtquelle 12 ist eine optoelektronische Empfangsvorrichtung 14 angeordnet, die den reflektierten Laserstrahl empfängt, der von einem relativ kleinen Retro-Reflektorelement 18 zurückgeworfen wird. Das Reflektorelement wird mit Hilfe geeigneter Mittel (hier nicht dargestellt) am Patienten, hier zum Beispiel an der Stirn eines Kopfes 20 des Patienten, befestigt, beispielsweise mit Hilfe eines Haflklebers. Der Kopf ist beispielsweise einer Strahlbehandlung unterworfen. Der vorzugsweise fest angeordnete Laser 12 erzeugt am Kopf 20 die Linie 16. Das Reflektorelement 18 wird im Bereich der Linie 16 aufgebracht, und die Empfangsvorrichtung 14 überwacht, ob das Reflektorelement die Linie 16 verläßt. Bewegt sich der Kopf 20 und damit das Reflektorelement 18, trifft der Laserstrahl nicht mehr das Reflektorelement. Damit ändert sich auch das Ausgangssignal der Empfangsvorrichtung 14, das zur Erzeugung eines Alarms optischer oder akustischer Natur verwendet werden kann. In der Zeichnung ist eine Signal-leitung 22 dargestellt, die zu einer optischen Anzeige 24 führt. Ferner dient sie zur Übertragung der Ein- und Aus-schaltsignale von der Betätigung 26 aus.

Die Vorrichtung 10 kann an beliebigen Orten angeordnet werden und wird mit Hilfe eines Netzkabels 28 mit Energie versorgt. Sie kann daher ohne weiteres an beliebigen Bestrahlungs- und Röntgenapparaturen angeordnet bzw. in diese integriert werden.

Die Größe des Reflektorelements 18 bestimmt die Ansprechempfindlichkeit der Vorrichtung. Je kleiner es ist, um so empfindlicher reagiert der Bewegungsmonitor.

## Patentansprüche

1. Vorrichtung zur Anzeige einer Bewegung eines Patienten mit einer einen Strahl erzeugenden Halbleiter-Lichtquelle sowie einer optoelektronischen Empfangsvorrichtung (14), die den von an einem Patienten angebrachten Retro-Reflektorelement (18) reflektierten Lichtstrahl empfängt und ein entsprechendes Signal erzeugt und mit der Empfangsvorrichtung (14) eine Anzeige und/oder Alarmvorrichtung (24) verbunden ist zur Erzeugung eines Anzeige- und/oder Alarmsignals, dadurch gekennzeichnet, daß die Lichtquelle ein Halbleiterlaser ist, der einen linienförmigen Strahl (16) erzeugt, der unmittelbar auf den Patienten (20) projiziert wird und daß der Halbleiterlaser (12) und die Empfangsvorrichtung (14) in einem gemeinsamen Gehäuse aufgenommen sind.

## Claims

1. A device for indicating the motion of a patient, the device comprising a semi-conductor light source generating a beam and an opto-electronic receiving means (14) which receives the light beam horn a retro-reflector element attached to the patient and generating a corresponding signal, an indicating and/or alarm means (24) being connected to the receiving means (14) for generating and/or alarm signal, characterized in that the light source is a semi-conductor laser which generates a line-shaped beam (16) which is directly projected onto the patient (20) and that the semi-conductor laser (12) and the receiving means (14) are accommodated in a common housing.

## Revendications

1. Dispositif pour l'affichage du mouvement d'un patient comportant une source de lumière à semi-conducteurs produisant un rayon, ainsi qu'un dispositif récepteur optoélectronique (14), qui reçoit le rayon lumineux réfléchi par un élément rétroréflecteur (18) fixé à un patient et produit un signal correspondant et un dispositif d'affichage et/ou d'alarme (24) est relié au dispositif récepteur (14) pour produire un signal d'affichage et/ou d'alarme, caractérisé en ce que la source de lumière est un laser à semi-conducteurs qui produit un rayon (16) en forme de ligne qui est projeté directement sur le patient (20), et en ce que le laser à semi-conducteurs (12) et le dispositif récepteur (14) sont logés dans un boîtier commun.
